# EUROPEAN PATENT SPECIFICATION

(11) **EP 4 461 237 B1**
(45) Date of publication and mention of the grant of the patent: **18.11.2026**
(21) Application number: 24203055.9
(22) Date of filing: 07.02.2020
(51) Int. Cl.: A61B 10/02, A61M 1/00, A61B 17/34, A61B 17/16

(54) **BONE MATERIAL HARVESTING DEVICE**
VORRICHTUNG ZUR GEWINNUNG VON KNOCHENMATERIAL
DISPOSITIF DE COLLECTE DE MATÉRIAU OSSEUX

(30) Priority: 13.02.2019 US 201962804987 P
(43) Date of publication of application: 13.11.2024
(62) Document number(s) of the earlier application(s) in accordance with Art. 76 EPC: 20723516.9 / 3 923 821
(73) Proprietor: Stryker European Operations Limited, Carrigtwohill, Co. Cork T45 HX08 (IE)
(72) Inventor: NASSUTT, Roman, 24143 Kiel (DE); BRÖHAN, Torben, 24103 Kiel (DE); GIERSCH, Helge, 24235 Laboe (DE); PRAGER, Ronald, 24796 Bovenau (DE)
(74) Representative: Maiwald GmbH

(56) References cited:
- WO-A1-99/39724
- US-A1- 2004 191 897
- US-A1- 2007 016 100
- US-A1- 2009 306 669
- US-A1- 2013 053 725
- US-A1- 2016 278 790

## Description

### CROSS-REFERENCE TO RELATED APPLICATIONS

This application claims the benefit of the filing date of United States Provisional Patent Application No. 62/804,987 filed February 13, 2019.

### BACKGROUND OF THE INVENTION

The present invention relates to devices for harvesting and processing bone and/or cellular material for use in various medical treatments.

Pseudarthrosis, also referred to as non-union of bone, is a common complication of fracture treatment. Non-union occurs when a particular patient's fracture site fails to heal within a specified period of time, and thus requires an intervention (e.g., surgical) in order to achieve proper union and mobility. In some cases, non-unions may be treated by bone grafting (e.g., allograft, autograft, or xenograft), through internal or external fixation, or a combination thereof. Bone grafting offers an opportunity to stimulate the fracture site so that bony formation occurs at the site to properly unionize the fracture.

Stem cells (e.g., Mesenchymal stem cells (MSCs), hematopoietic stem cells (HSCs), or other stem cells) are known to be useful with certain graft materials, or by themselves, to facilitate bone growth and formation when used appropriately. For example, adult MSCs are capable of differentiating into a variety of different cell types including osteoblasts (bone cells), chondrocytes (cartilage cells), and adipocytes (fat cells). As such, when incorporated with certain allograft material, stem cells can assist with the proper formation of bone and union of bone parts at a fracture site. Stem cells of the type discussed above must first be extracted from a patient and processed before use. As an example, extracted bone marrow of a patient can provide the necessary stem cells.

Bone marrow is typically extracted in a multi-staged procedure. In a first stage of the procedure, bone marrow is aspirated from a patient and then sent to a laboratory for processing. It is only then, in a second stage of the procedure, that the previously harvested bone marrow is processed for the patient's particular application. Bone marrow from the iliac crest is widely considered the "gold standard" for its superior biologic quality. The drawback of harvesting bone marrow from the iliac crest, however, is that the iliac crest naturally produces a limited quantity of marrow. For surgical interventions requiring a higher volume of bone marrow, such as trauma and orthopedic applications, bone marrow is often harvested from the medullary canal of a long bone (e.g., the femur or tibia), which naturally produces a higher volume of bone marrow than the iliac crest. Bone marrow harvested from the medullary canal has proven to be an effective alternative to bone marrow harvested from the iliac crest and is capable of providing significant regenerative potential.

In order to harvest the desired volume of marrow from the medullary canal, a surgeon must access and aspirate bone marrow located deep within the bone canal. This procedure is often performed using a harvesting system that is typically equipped with a reamer, an irrigation system, a suction source

(OR suction) and a filter. Although these systems are generally sufficient in harvesting the desired bone material, they are aggressive and carry significant clinical risks such as severe blood loss that often necessitates blood transfusion treatments. In fact, while the medical community appreciates the regenerative potential of bone marrow harvested from the medullary canal of long bone, some medical professionals have begun to question whether harvesting the autograft materials is worth the significant clinical risks. US 2004/191897 A1 discloses an apparatus for harvesting bone marrow cells, blood and bone fragments used in transplantation, has rigid cannula, and unit for drawing bone marrow cells by cutting tip into passage for collection.

Known harvesting devices are also susceptible to clogging, which results in decreased aspiration function, especially while positioned deep within the medullary canal. Moreover, the harvested material is often diluted and/or contaminated by the water or other solution introduced by the irrigation system. Such dilution or contamination complicates the processing stage of the procedure and necessitates additional filtering or refining of the harvest. Therefore, new and improved bone harvesting devices that address these shortcomings are desired.

### BRIEF SUMMARY OF THE INVENTION

In accordance with a first aspect of the present invention, a device for harvesting bone and/or cellular material (e.g., cancellous bone, bone chips, bone marrow, and stem cells) for use in various medical applications is provided. Among other advantages, the device includes a dual tubed aspiration assembly that reduces clogging and improves aspiration efficiency. In part, due to the improved aspiration efficiency, the device is capable of performing clog-free transportation of even highly viscous material without the irrigation of fluids. As a result, the need for subsequent filtering and excessive processing is eliminated. Furthermore, the improved aspiration function reduces the surgeon's impulse to aggressively drill into the bone and, in turn, reduces the likelihood of severe blood loss and the clinical complications associated with sever blood loss.

The invention is defined by claim 1. Further embodiments of the invention are defined by the dependent claims.

One embodiment of the bone harvesting device includes a main body having an internal cavity and a suction port adapted to be coupled to a suction source, an outer tube having an inner surface, an outer surface, a proximal end and a distal end, the proximal end of the outer tube being coupleable to the main body, an inner tube having an inner surface, an outer surface, a proximal end and a distal end, the inner tube being coupleable to the main body and at least partially disposed within the outer tube such that the inner tube is in fluid communication with the internal cavity, and a storage container coupleable to the main body such that the storage container is in fluid communication with the internal cavity for receiving bone and/or cellular material extracted from the patient.

In some embodiments, the storage container includes an outer sidewall extending from a base end to a top end, and an inner sidewall at least partially disposed within the outer sidewall and being concave with respect to the top end, the inner sidewall defining a collection space for receiving the extracted bone and/or cellular material. At least a portion of the inner sidewall is spaced a distance from the outer sidewall.

In another embodiment, the bone harvesting device may include a body having an internal cavity and a suction port adapted to be coupled to a suction source, an outer tube having an inner surface, an outer surface, a proximal end and a distal end, the proximal end of the outer tube being coupleable to the body, and an inner tube having an inner surface, an outer surface, a proximal end and a distal end. The inner tube may be in fluid communication with the internal cavity and at least partially disposed within the outer tube such that a venting channel is defined between at least a portion of the inner tube and at least a portion of the outer tube.

A method of collecting bone and/or cellular material during a bone harvesting procedure is also provided herein. The method includes the steps of (a) operating a reaming, milling, or drilling tool so that the tool reams, mills, or drills into a canal of a bone to generate bone and/or cellular material, (b) inserting a bone harvesting tool into the bone canal, the bone harvesting tool comprising an inner tube at least partially disposed within an outer tube such that a ventilation channel is formed between the inner and outer tubes, (c) generating negative pressure within an internal cavity of a bone harvesting tool, the negative pressure causing the bone and/or cellular material to flow from the patient through the inner tube and into the internal cavity of the bone harvesting tool, and collecting the bone and/or cellular material in a storage container fluidly connected to the internal cavity of the bone harvesting tool.

### BRIEF DESCRIPTION OF THE DRAWINGS

FIG. 1 is a perspective view of a bone harvesting device according to an embodiment of the invention.
FIG. 2 is an exploded view of the bone harvesting device of FIG. 1.
FIG. 3 is a partial cross-section of the bone harvesting device of FIG. 1 taken along line A-A.
FIG. 4A is a side view of an aspiration assembly of the bone harvesting device of FIG. 1.
FIG. 4B is an enlarged, partial cross-section view of the proximal end of the aspiration assembly of FIG. 4A taken along line B-B.
FIG. 4C is an enlarged, partial cross-section view of the distal end of the aspiration assembly of FIG. 4 taken along line B-B.
FIG. 4D is a partial, front-oriented perspective view of the aspiration assembly of FIG. 4A.
FIGS. 5A-5F are front plan views illustrating exemplary arrangements of the aspiration assembly.
FIG. 6 is a perspective view of the collection storage container of FIG. 1.
FIG. 7 is a cross-section view of the collection storage container of FIG. 6 taken along line C-C.
FIG. 8 is flow chart illustrating a method of harvesting bone and/or cellular material from a patient.
FIG. 9 is a schematic representation illustrating the bone harvesting device of FIG. 1 aspirating bone and/or cellular material from a patient's femur.
FIG. 10 is a schematic representation illustrating a known bone harvesting device aspirating bone and/or cellular material from a patient's femur.

### DETAILED DESCRIPTION

As used herein, "axial" means along or parallel to the longitudinal axis of the bone harvesting device and "radial" means in the perpendicular direction thereto. "Interior" or "inner" means radially inward, either toward or facing the longitudinal axis, and "exterior" or "outer" means radially outward, or away from the longitudinal axis. The terms "proximal" and "distal" refer, respectively, to the end of the device nearest the surgeon or other user operating the device, and the opposite end of the device furthest from the user operating the device.

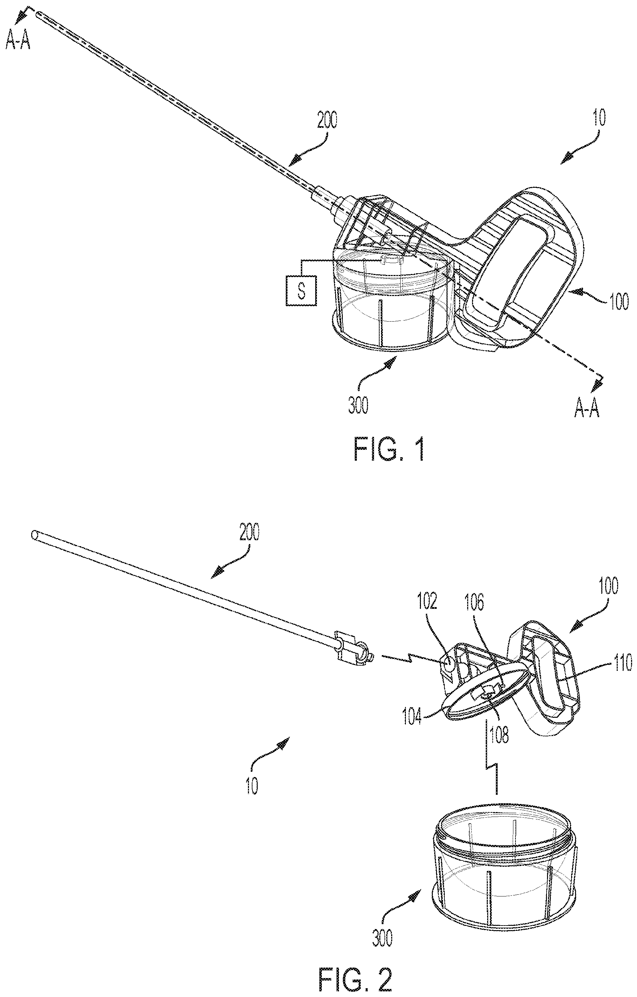

Bone harvesting device 10, as shown in FIGS. 1 and 2, includes a body 100, an aspiration assembly 200 for aspirating bone and/or cellular material from a patient, and a collection storage container 300 for collecting the aspirated material. The phrase bone and/or cellular material refers to material that is extractable from bone, which may optionally then be processed and/or separated to produce another material. For instance, bone and/or cellular material may include cancellous bone, cortical bone (in the form of chips or morselized bone), bone marrow, or stem cells produced from any of the foregoing materials. Such materials are frequently found, for example, in the iliac crest, or the medullary canal of a long bone, as well as the bone surrounding the medullary canal. Bone harvesting device 10 may be formed entirely, or in part, from a transparent medical grade glass or plastic, thereby permitting an operator using the device to observe the collection of bone and/or cellular material during a harvesting procedure.

Main body 100 includes a first connector, for example, a threaded bore 102, for removably securing aspiration assembly 200 to the body, and a second connector, for example, a threaded, lid-shaped platform 104 for removably securing storage container 300 to the body. It will be appreciated, however, that the first and second connectors need not include threads. Instead, aspiration assembly 200 and storage container 300 may alternatively be coupled to the main body 100 via an interference or snap-fit connection, or any other connector known in the art. Main body 100 also defines a suction port 106, for example, in lid-shaped platform 104 that is configured to be coupled to a suction source S such as a standard operating room suction source (OR suction). Suction port 106 may include a filter to prevent bone and/or cellular material from being drawn from storage container 300 into a suction tube connected suction source S.

With specific reference to FIG. 2, platform 104 has an aperture 108 that allows harvested bone and/or cellular material to flow from aspiration assembly 200 and into storage container 300 during a harvesting procedure. As shown, main body 100 may also include an ergonomically shaped handle 110 to aid a surgeon in positioning the bone harvesting device during operation.

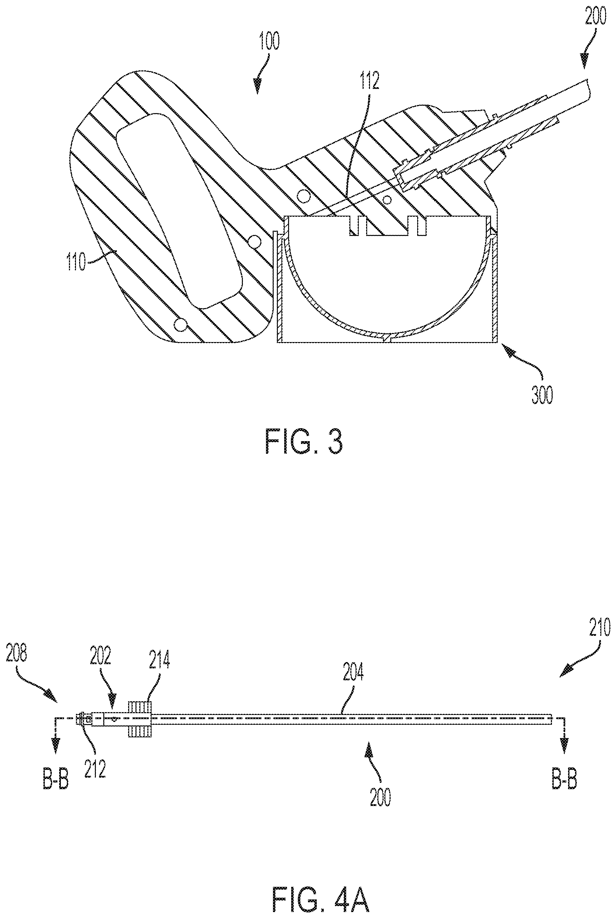

Referring to FIG. 3, main body 100 defines an internal cavity 112 that is in fluid communication with aspiration assembly 200 and suction source S. Thus, when OR suction is applied, a negative pressure is produced within internal cavity 110. This negative pressure draws bone and/or cellular material through aspiration assembly 200, into the internal cavity, through the aperture 108 and into storage container 300.

Aspiration assembly 200, as shown in FIGS. 4A-4D, includes a generally hollow adapter housing 202, an outer tube 204 and an inner tube 206. Outer tube 204 and inner tube 206 are formed of a resilient and flexible material that allows the tubes to bend, with respect to the main body 100 and with respect to one another, as the aspiration assembly navigates through the medullary canal of a long bone. Aspiration assembly 200 defines a length between a proximal end 208 and a distal end 210 of the assembly. Bone harvesting device 10 may include a single aspiration assembly 200 measuring approximately 500 mm in length, or a plurality of interchangeable aspiration assemblies having various lengths for harvesting bone of different length.

The proximal end 208 of aspiration assembly 200 includes threads 212 for threading adapter 202 within the bore 102 of main body 100. Adapter 202 need not include threads, however, as aspiration assembly 200 may instead be removably coupled to main body 100 by an interference or snap fit connection, or any other connector known in the art. Adaptor 202 may also include a flange 214, or similar protrusion having an ergonomic grip to aid a user in coupling and/or decoupling the aspiration assembly 200 to the main body 100.

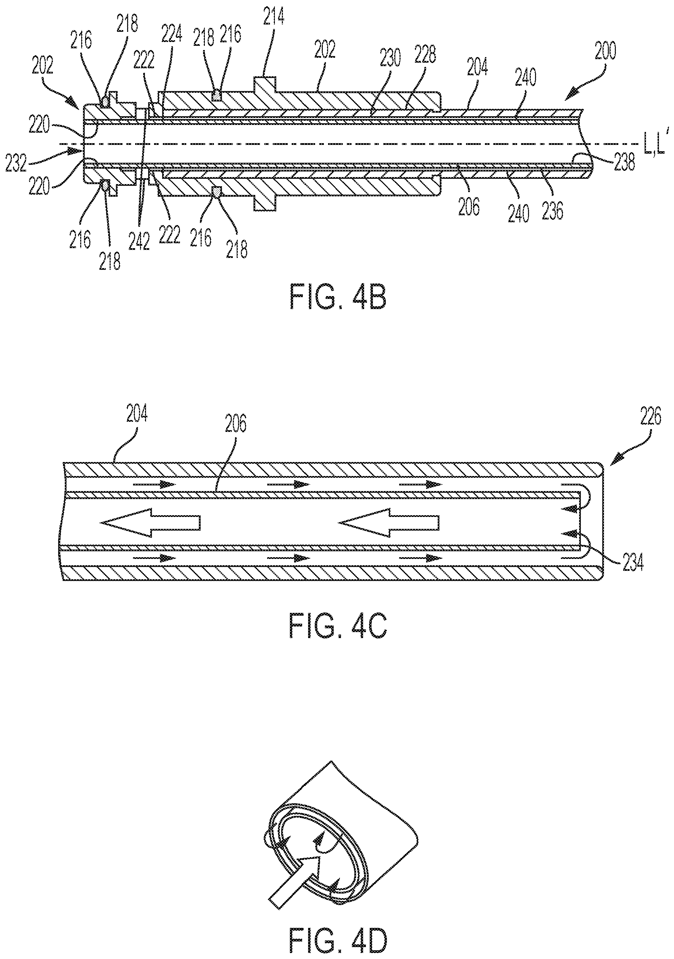

With specific reference to FIG. 4B, adaptor 202 has one or more annular grooves 216 within its exterior surface. A gasket 218, such as an O-ring, is provided within each groove 216 to seal the space between the adaptor 202 and the bore 102 of the main body at each location. Adapter 202 also includes first and second inwardly projecting ledges 220, 222. The first ledge 220 is provided adjacent the proximal end of adapter 202, while the second ledge 222 is positioned between the first ledge and the flange 214. First ledge 220 protrudes further into the interior space of the adapter 202 than the second ledge 222.

Outer tube 204 extends along a longitudinal axis L from a proximal end 224 to a distal end 226 and includes an exterior surface 228 and an interior surface 230. The exterior surface 228 of outer tube 204 engages the interior surface of adapter 202, while the proximal end 224 of the outer tube is bonded, welded, glued or otherwise sealed to a distal facing surface of the second ledge 222. The distal end 226 of outer tube 204 is preferably rounded, as shown in FIG. 4C, to reduce trauma as aspiration assembly 200 is operated within the medullary canal of bone.

Inner tube 206, which is positioned at least partially within outer tube 204, extends along a longitudinal axis L' from a proximal end 232 to a distal end 234 and includes an exterior surface 236 and an interior surface 238. Inner tube 206 is bonded, welded, glued or otherwise sealed to an inwardly facing surface of the first ledge 220, thereby forming a venting channel 240 between the inner and outer tubes.

Adapter 202 further defines a vent 242 axially located between the first and second ledges 220, 222. Vent 242 is in fluid communication with ambient air and venting channel 240. As shown in FIGS. 4C and 4D, this allows ambient air drawn into vent 242 to flow through venting channel 240 and into the medullary canal adjacent the distal end of the aspiration assembly, while bone and/or cellular material is concurrently aspirated through the inner tube 206 in the opposite direction (e.g., from the distal end of the aspiration assembly toward the main body). The ventilated air provides airflow through the medullary canal during the harvesting process and prevents the medullary canal from becoming negatively pressurized during aspiration and, as a result, reduces the amount of blood loss that occurs during bone and/or cellular aspiration as is further explained hereinafter.

**In** some embodiments, the distal end 234 of inner tube 206 may be proximal to the distal end 226 of outer tube 204. The vented air is thus drawn around the distal end 234 of inner tube 206 and into the aspiration channel of the inner tube. This reduces clogging at the distal end of the aspiration assembly and maintains optimal suction or aspiration power. Moreover, because inner tube 206 is surrounded by the outer tube 204, the inner is protected from being damaged.

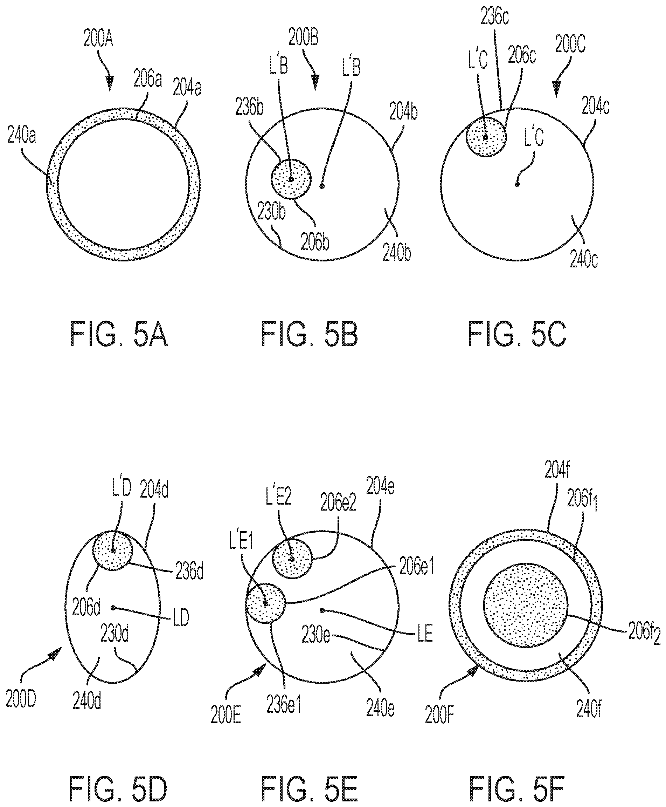

Referring to FIGS. 5A-5F, various exemplary configurations of the aspiration assembly are described. FIG. 5A illustrates a preferred aspiration assembly 200A, in which inner tube 206a is coaxially positioned within outer tube 204a. Venting channel 240a is thus formed around the entire exterior surface of inner tube 206a.

FIG. 5B shows aspiration assembly 200b, in which the longitudinal axis L'_{B} of inner tube 206b is offset with respect to the longitudinal axis L_{B} of outer tube 204b, and the exterior surface 236b of the inner tube is spaced from the interior surface 230b of the outer tube. Venting channel 240b entirely surrounds inner tube 206b.

FIG. 5C illustrates a third configuration, aspiration assembly 200c, in which the longitudinal axis L'_{C} of inner tube 206c is offset with respect to the longitudinal axis L_{C} of outer tube 204c. In this configuration, however, a portion of the exterior surface 236c of inner tube 206c engages a portion of the interior surface 230c of outer tube 204c. Venting channel 240c is thus only formed around a portion of inner tube 206c (i.e., the portion of the exterior surface of the inner tube that is not engaged with the outer tube).

FIG. 5D illustrates aspiration assembly 200d, which is similar to aspiration assembly 200c, in that the longitudinal axis L'_{D} of inner tube 206d is offset with respect to the longitudinal axis L_{D} of outer tube 204d, and in that a portion of the exterior surface 236d of the inner tube is engaged with a portion of the interior surface 230d of the outer tube such that venting channel 240d is formed only around a portion of the inner tube. In this configuration, however, the cross-section of outer tube 204d is elliptical. It will be appreciated that the inner tube or the outer tube, or both the inner tube and the outer tube, of any of the configurations shown in FIGS. 5A-5F, may have a cross-section forming an ellipse, a rectangle, a hexagon or any other shape.

Aspiration assembly 200e, as shown in FIG. 5E, includes first and second inner tubes 206e₁, 206e_{2.} The longitudinal axis L'_{E1} of first inner tube 206e₁ and the longitudinal axis L'_{E2} of second inner tube 206e₂ are offset with respect to the longitudinal axis L_{E} of outer tube 204e. A portion of the exterior surface 236e₁ of first inner tube 206e₁ and a portion of the exterior surface 236e₂ of second inner tube 206e₂ are engaged with portions of the interior surface 230e of outer tube 204e. Venting channel 240e is thus formed only around the portions of inner tubes 206e₁, 206e₂ that are not engaged with outer tube 204e. It will be understood, however, that one or both of inner tubes 206e₁, 206e₂ may instead be spaced from the interior surface 230e of outer tube 204e such that venting channel 240e is formed entirely around the inner tube, or the inner tubes, spaced from the interior surface of the outer tube.

FIG. 5F illustrates aspiration assembly 200f. In this configuration, first inner tube 206f₁ is coaxially positioned within outer tube 204f and second inner tube 206f₂ is coaxially positioned within the first inner tube. Venting channel 240f is thus positioned between the first and second inner tubes 206f₁, 206f₂.

It will be appreciated that aspiration assembly 200 may be constructed as described in any one of the embodiments shown in FIGS. 5A-5F or as a combination of the same. Regardless of the specific construction, it is generally desirable that the aspiration channel (e.g., the opening of the inner tube(s)) has a cross-sectional area of at least 12 mm². Moreover, to ensure that sufficient air is vented into the medullary canal, it is desirable that the ratio of the surface area of the cross-section of the venting channel relative to the surface area of the cross-section of the aspiration channel be between 0.15 and 1.0. It will be understood that FIGS. 5A-5F illustrate the inner and outer tubes in their unbent, or not flexed, condition. Any bending or flexing of inner tube 206, relative to outer tube 204, will change the location of the aspiration channel and, in turn, venting channel 240 relative to the outer tube, but will not modify the cross-sectional area of the aspiration channel or the cross sectional area of the venting channel.

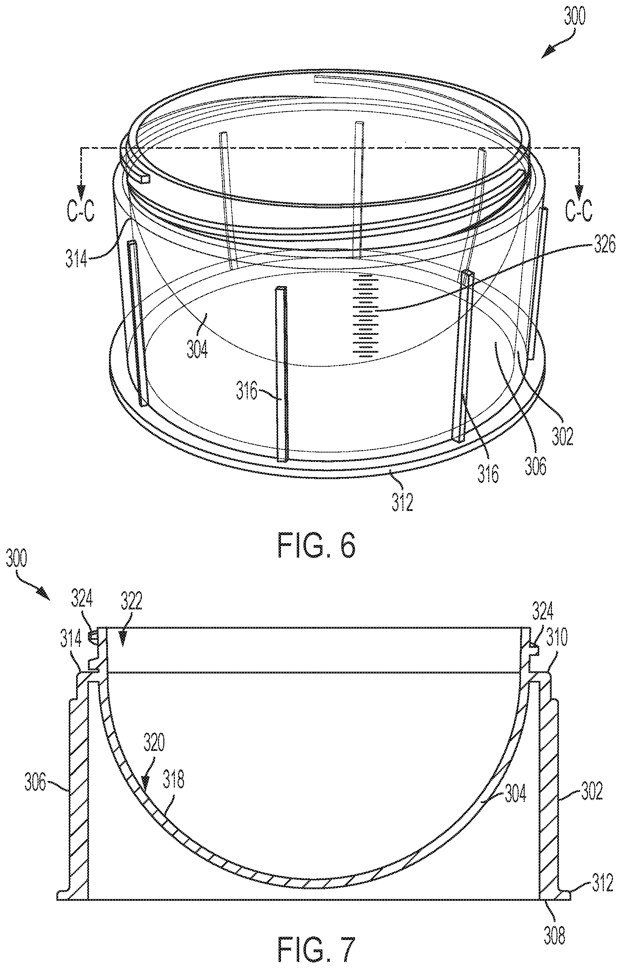

Turning now to FIGS. 6 and 7, storage container 300 includes a stand 302 and a collection bowl 304. Storage container 300 is preferably formed as an integral component of a plastic material, for example, through an injection molding process. Alternatively, stand 302 and collection bowl 304 may be coupled together after the components are separately manufactured.

Stand 302 may include a cylindrical sidewall 306 that extends from an annular base 308 to a top end 310 and that defines a diameter and height. Base 308 is planar and, thus, configured to rest on a flat surface, for example, an operating table. In a preferred embodiment, the diameter of stand 302 is at least 1.5 times greater than the height to save stance on soft and irregular surfaces. Base 308 may include a flange 312 for added stability.

Top end 310 preferably includes an inwardly extending lip 314 for receiving a gasket (not shown) to seal the connection between container 300 and main body 100. Sidewall 306 may include a plurality of ergonomically shaped grips 316 to aid a user in connecting the storage container 300 to the main body 100 and for removing the container from the body. Grips 316 are also thickened areas of material that strengthen sidewall 306.

Collection bowl 304 includes a sidewall 318 that is connected to lip 314 such that a first portion 320 of the collection bowl sits within the sidewall 306 of stand 302 and a second portion 322 of the collection bowl extends above the top end 310 of the stand.

As shown, the second portion 322 includes threads 324, for example, steep or bayonet threads for coupling the storage container 300 to the lid-shaped platform 104 of the main body. Collections bowl 304 may alternatively include any connector capable of removably coupling the storage container and the main body.

The inner surface of sidewall 318 is preferably smooth and hemispherical in shape. In contrast to an ordinary container having a flat base and a sidewall, the interior surface of hemispherical collection bowl 304 is devoid of edges. Because bone and/or cellular material is harvested in relatively small and finite quantities, and through an invasive surgical procedure, it is desirable to preserve all of the material that is harvested from the patient for subsequently processing. The hemispherical shape of the collection bowl 304 assists in this effort as it allows a user to easily remove all of the highly viscous bone and/or cellular material without requiring that the user attempt to scrape material from the edges or the corners of the container before inevitably leaving traces of the material behind. Thus, the hemispherical collection bowl 304 saves the user both time and hassle and maximizes the bone and/or cellular material that is eventually processed. To aid the user in removing bone and/or cellular material from collection bowl 304, the open end of sidewall 318 is preferably at least 80 mm in diameter, thereby allowing the user to easily fit his or her hand into the collection bowl and to scoop out the material, for example, using his or her fingers.

**In** some embodiments, an indicator scale 326 may be molded to, or imprinted on, storage container 300 such that the user can easily determine the volume of bone and/or cellular material that is present within collection bowl 304.

Bone harvesting device 10 may be used to harvest bone and/or cellular material in a harvesting method as provided herein. The method generally includes extracting bone and/or cellular material from a patient and then sending it to a storage, separation and processing facility (e.g., a "biobank") for use in a later surgical procedure involving that patient, or a different patient.

**In** many instances, a patient is scheduled to undergo a surgical procedure that necessitates resection of bone and/or reaming of a medullary canal of bone, but in which harvesting bone and/or cellular material is not the primary objective of the surgical procedure. For example, intramedullary nailing (IM nail) procedures, hip replacements, and knee revisions, each require a substantial amount of resection and/or reaming of bone, and therefore, generate bone and/or cellular material as a byproduct of the surgery.

During an **IM** nail procedure, for example, a surgeon seeking to remedy a fracture in the tibia typically first makes an incision in the patient's skin adjacent the knee. A K-wire is then introduced through the patient's skin and into an entry point on the patient's tibial plateau. In a reamed technique, the surgeon then uses the K-wire to guide a reamer through the entry point and into the medullary canal of the long bone. With the K-wire extending into the long bone and past the fracture site (e.g., so that the fracture can be properly reduced), one or more reamers are then used to bore through cortical and cancellous bone, as well as bone marrow of the patient. Because harvesting bone and/or cellular material is not the primary objective of the IM nail procedure, and extraction of these materials can often result in sever blood loss and other complication, bone and/or cellular material is generally only extracted from the medullary canal of the tibia to the extent necessary to make room for the IM implant. Any cortical bone, cancellous bone and bone marrow material that is extracted during the reaming procedure is often discarded. Using harvesting device 10, however, bone and/or cellular materials can be safely extracted from the patient for subsequent processing and reuse without placing the patient at risk.

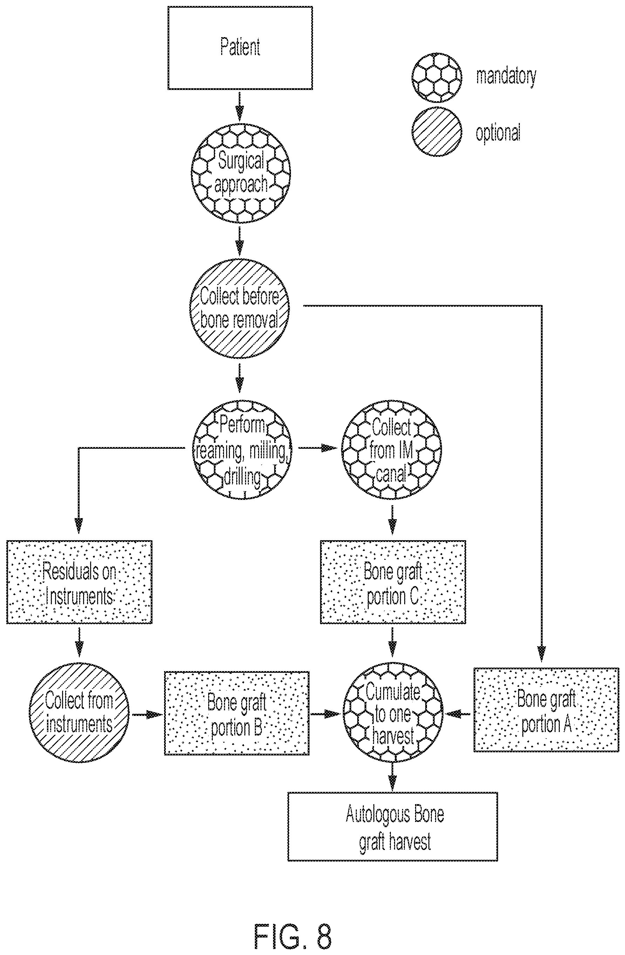

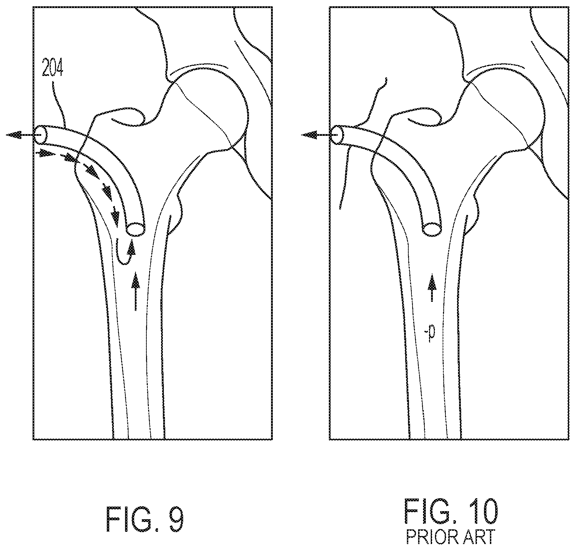

Referring to FIGS. 8 and 9, a method of extracting bone and/or cellular material from a patient is described, using an IM nail procedure remedying a patient's fractured femur as an illustrative example. It will be understood, however, that bone harvesting device 10 significantly reduces surgical complications when used to aspirate bone and/or cellular material within a medullary cavity and, thus, may likewise be used to harvest material from within the medullary canal of the tibia or any other bone from which bone and/or cellular material may be harvested.

In reducing a fractured femur, a surgeon may first approach the fracture by making an incision in the patient's skin adjacent the hip. The surgeon may then resect the greater trochanter, thereby creating an opening to the medullary canal of the femur. Resection of the greater trochanter generates loose cortical and cancellous bone, which may optionally be collected, either by hand or using bone harvesting device 10. To collect the loose cortical and cancellous bone using harvesting device 10, a user turns on the OR suction S, which generates a negative pressure in the internal cavity 112 of main body 10. Using handle 110, the user may position the distal end 210 of the aspiration assembly adjacent the resected bone, causing the loose cortical and cancellous bone to be drawn through the aspiration channel of inner tube 206, into the internal cavity, through aperture 108 in lid 104 and into storage container 300. After the loose cortical and cancellous bone has been collected, the user may turn off the OR suction S.

A K-wire may then be introduced through the patient's skin and into the medullary canal of the femur. The surgeon may then use the K-wire to guide one or more reaming, milling or drilling tools through an entry point and into the medullary canal of the femur. With the K-wire extending within the medullary canal and past the fracture site, the tool is used to bore through cortical and cancellous bone, as well as bone marrow of the patient, generating bone and/or cellular material in the medullary canal. After the surgeon is satisfied that a bore of sufficient size has been created to receive the IM implant, the tool may be removed from the patient. The surgeon may then optionally collect the residual bone and/or cellular material that adhered to the tool(s) during the reaming, milling or drilling of the medullary canal.

Before implanting the IM nail, the surgeon may utilize harvesting device 10 to safely collect bone and/or cellular material from the medullary canal of the femur. In doing so, the OR suction S is again activated. With a negative pressure generated in the internal cavity 112 of main body 110, the surgeon may position the distal end 210 of the aspiration assembly into the medullary canal of the femur such that the main body is positioned outside the patient.

Referring to FIG. 9, schematically illustrating only inner tube 206 for clarity, ambient air enters vent 242 (FIG. 4B) and flows through venting channel 240 (FIG. 4c) and into the medullary canal. At the same time, the negative pressure generated within the internal cavity 112 of main body 100 draws bone and/or cellular material through the aspiration channel of inner tube 206 and into storage container 300. Because air is continually vented within the medullary canal adjacent the distal end 210 of aspiration assembly 200, the medullary canal does not become negatively pressurized during aspiration of the bone and/or cellular material. Moreover, in the embodiment in which the distal end 234 of inner tube 206 is provided proximal to the distal end 226 of outer tube 204, the vented air is drawn around the distal end of the inner tube and into the aspiration channel thereof. The curling of the air adjacent the distal end 210 of aspiration assembly 200 guides bone and/or cellular material into the aspiration channel of inner tube 206 such that clogging is reduced and optimal suction or aspiration power is maintained.

In contrast, as is shown in FIG. 10, illustrating a known bone harvesting device that does not include the described venting features, as bone and/or cellular material is aspirated though the aspiration assembly, the medullary canal becomes negatively pressurized. The negative pressure results in the aspiration of blood and can result in sever blood loss. Additionally, as the medullary canal drops in pressure, the aspiration power of the assembly is reduced, resulting in decreased aspiration efficiency and often clogging of the assembly.

Referring back to FIG. 8, the user may optionally combine the bone and/or cellular materials into a cumulate harvest. In order to do so, the user first uncouples storage container 300 from main body 100, sticks his or her hand into collection bowl 304 and scoops out all of the bone and/or cellular material. The bone and/or cellular material may then be combined with the residual bone and/or cellular material collected from the reaming, milling or drilling tools and/or the loose bone and/or cellular material generated during the bone resection step.

After the bone and/or cellular material has been harvested, the surgeon may implant the IM nail and finish the IM nail procedure.

The bone and/or cellular material may then be sent to a biobank as separate collections, or as a cumulate harvest, for subsequent processing and use in a later surgical procedure involving that patient, or a different patient.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised. The invention is defined by the appended claims.

## Claims

1. A bone harvesting device (10) for extraction of bone and/or cellular material from a patient during a surgical procedure, the device comprising:
a main body (100) defining an internal cavity (112), the main body including a suction port (106) adapted to be coupled to a suction source (S);
an outer tube (204) having a proximal end (224) coupleable to the main body (100) and a distal end (226); and
an inner tube (206) at least partially disposed within the outer tube (204) and having a proximal end (232) and a distal end (234),
wherein a venting channel (240) is defined between at least a portion of the inner tube (206) and at least a portion of the outer tube (204) such that ambient air flows through the venting channel (240) in a proximal-distal direction and curls around the distal end (234) of the inner tube (206) while the bone and/or cellular material is aspirated through the inner tube (206) in a distal-proximal direction.

2. The device (10) of claim 1, further comprising a suction source (S) fluidly connected with the main body (110) and adapted to generate negative pressure within the internal cavity (112) of the main body.

3. The device (10) of claim 1 or 2, further comprising a hollow adapter housing (202) in which the proximal ends of the inner and outer tubes are received, the adapter housing having a first inwardly projecting ledge (220), an exterior surface (236) of the inner tube (206) forming a seal with the first inwardly projecting ledge.

4. The device (10) of claim 3, wherein the venting channel (240) extends from the seal to the distal end (234) of the inner tube (206).

5. The device (10) of one of the preceding claims 3 or 4, wherein the inner and outer tubes are coaxially positioned such that the venting channel (240) surrounds the exterior surface (236) of the inner tube (206).

6. The device (10) of one of the preceding claims 3 to 5, wherein the adapter housing (202) defines at least one vent (242) in fluid communication with the venting channel (240).

7. The device (10) of one of the preceding claims 3 to 6, wherein the adapter housing (202) comprises a thread (212).

8. The device (10) of any of the preceding claims, wherein the distal end (234) of the inner tube (206) is located proximal to the distal end (226) of the outer tube (204).

9. The device (10) of any of the preceding claims, wherein the distal end (226) of the outer tube (204) includes a rounded edge.

10. The device of any of the preceding claims, further comprising a storage container (300) coupleable to the main body (100) and in fluid communication with the internal cavity (112) for receiving bone and/or cellular material extracted from the patient.

11. The device of one of the preceding claims 3 to 10, wherein the adapter housing (202) further includes a second inwardly projecting ledge (222) distal to the first ledge (220), the proximal end (224) of the outer tube (204) being engaged with the second inwardly projecting ledge.

12. The device of the preceding claim, wherein the at least one vent (242) is axially located between the first and second ledges.

13. The device of any of the preceding claims, wherein the inner tube (206) is in fluid communication with the internal cavity (112).

14. The device of any of the preceding claims, wherein the venting channel is defined between a portion of an exterior surface (236) of the inner tube (206) and an interior surface (230) of the outer tube (204).

## Patentansprüche

1. Eine Knochenentnahmevorrichtung (10) zum Extrahieren von Knochen- und/oder Zellmaterial von einem Patienten während eines chirurgischen Eingriffs, die Vorrichtung umfassend:
einen Hauptkörper (100), der einen inneren Hohlraum (112) definiert, wobei der Hauptkörper einen Sauganschluss (106) beinhaltet, der angepasst ist, um an eine Saugquelle (S) gekoppelt zu werden;
ein Außenrohr (204) mit einem proximalen Ende (224), das an den Hauptkörper (100) gekoppelt werden kann, und einem distalen Ende (226); und
ein Innenrohr (206), das zumindest teilweise innerhalb des Außenrohrs (204) angeordnet ist und ein proximales Ende (232) und ein distales Ende (234) aufweist,
wobei ein Ventilationskanal (240) zwischen mindestens einem Abschnitt des Innenrohrs (206) und mindestens einem Abschnitt des Außenrohrs (204) definiert ist, sodass Umgebungsluft durch den Ventilationskanal (240) in einer proximal-distalen Richtung strömt und um das distale Ende (234) des Innenrohrs (206) wirbelt, während das Knochen- und/oder Zellmaterial durch das Innenrohr (206) in einer distal-proximalen Richtung angesaugt wird.

2. Vorrichtung (10) nach Anspruch 1, ferner umfassend eine Saugquelle (S), die fluidisch mit dem Hauptkörper (110) verbunden ist und angepasst ist, um einen Unterdruck innerhalb des inneren Hohlraums (112) des Hauptkörpers zu erzeugen.

3. Vorrichtung (10) nach Anspruch 1 oder 2, ferner umfassend ein hohles Adaptergehäuse (202), in dem die proximalen Enden des Innen- und Außenrohrs aufgenommen sind, wobei das Adaptergehäuse einen ersten nach innen vorstehenden Vorsprung (220) aufweist, wobei eine Außenfläche (236) des Innenrohrs (206) eine Dichtung mit dem ersten nach innen vorstehenden Vorsprung bildet.

4. Vorrichtung (10) nach Anspruch 3, wobei sich der Ventilationskanal (240) von der Dichtung zu dem distalen Ende (234) des Innenrohrs (206) erstreckt.

5. Vorrichtung (10) nach einem der vorhergehenden Ansprüche 3 oder 4, wobei das Innen- und das Außenrohr koaxial positioniert sind, so dass der Ventilationskanal (240) die Außenfläche (236) des Innenrohrs (206) umgibt.

6. Vorrichtung (10) nach einem der vorhergehenden Ansprüche 3 bis 5, wobei das Adaptergehäuse (202) mindestens eine Belüftungsöffnung (242) in Fluidkommunikation mit dem Ventilationskanal (240) definiert.

7. Vorrichtung (10) nach einem der vorhergehenden Ansprüche 3 bis 6, wobei das Adaptergehäuse (202) ein Gewinde (212) umfasst.

8. Vorrichtung (10) nach einem der vorhergehenden Ansprüche, wobei das distale Ende (234) des Innenrohrs (206) proximal zu dem distalen Ende (226) des Außenrohrs (204) angeordnet ist.

9. Vorrichtung (10) nach einem der vorhergehenden Ansprüche, wobei das distale Ende (226) des Außenrohrs (204) eine abgerundete Kante beinhaltet.

10. Vorrichtung nach einem der vorhergehenden Ansprüche, ferner umfassend einen Aufbewahrungsbehälter (300), der an den Hauptkörper (100) gekoppelt werden kann und in Fluidkommunikation mit dem inneren Hohlraum (112) steht, um Knochen- und/oder Zellmaterial aufzunehmen, das von dem Patienten extrahiert wurde.

11. Vorrichtung nach einem der vorhergehenden Ansprüche 3 bis 10, wobei das Adaptergehäuse (202) ferner einen zweiten nach innen vorstehenden Vorsprung (222) distal zu dem ersten Vorsprung (220) beinhaltet, wobei das proximale Ende (224) des Außenrohrs (204) mit dem zweiten nach innen vorstehenden Vorsprung in Eingriff steht.

12. Vorrichtung nach dem vorhergehenden Anspruch, wobei die mindestens eine Belüftungsöffnung (242) axial zwischen dem ersten und dem zweiten Vorsprung angeordnet ist.

13. Vorrichtung nach einem der vorhergehenden Ansprüche, wobei das Innenrohr (206) in Fluidkommunikation mit dem inneren Hohlraum (112) steht.

14. Vorrichtung nach einem der vorhergehenden Ansprüche, wobei der Ventilationskanal zwischen einem Abschnitt einer Außenfläche (236) des Innenrohrs (206) und einer Innenfläche (230) des Außenrohrs (204) definiert ist.

## Revendications

1. Dispositif de prélèvement osseux (10) destiné à extraire du matériau osseux et/ou cellulaire d'un patient au cours d'une intervention chirurgicale, le dispositif comprenant :
un corps principal (100) définissant une cavité interne (112), le corps principal comportant un orifice d'aspiration (106) conçu pour être couplé à une source d'aspiration (S) ;
un tube extérieur (204) présentant une extrémité proximale (224) pouvant être couplée au corps principal (100) et une extrémité distale (226) ; et
un tube intérieur (206) disposé au moins partiellement au sein du tube extérieur (204) et présentant une extrémité proximale (232) et une extrémité distale (234),
dans lequel un canal de mise à l'air (240) est défini entre au moins une partie du tube intérieur (206) et au moins une partie du tube extérieur (204), de telle sorte que de l'air ambiant circule à travers le canal de mise à l'air (240) dans une direction proximale-distale et s'incurve autour de l'extrémité distale (234) du tube intérieur (206), tandis que le matériau osseux et/ou cellulaire est aspiré à travers le tube intérieur (206) dans une direction distale-proximale.

2. Dispositif (10) selon la revendication 1, comprenant en outre une source d'aspiration (S) reliée fluidiquement au corps principal (110) et adaptée pour générer une pression négative au sein de la cavité interne (112) du corps principal.

3. Dispositif (10) selon la revendication 1 ou 2, comprenant en outre un boîtier d'adaptateur creux (202) dans lequel sont reçues les extrémités proximales des tubes intérieur et extérieur, le boîtier d'adaptateur présentant un premier épaulement faisant saillie vers l'intérieur (220), une surface extérieure (236) du tube intérieur (206) formant un joint étanche avec le premier épaulement faisant saillie vers l'intérieur.

4. Dispositif (10) selon la revendication 3, dans lequel le canal de mise à l'air (240) s'étend du joint étanche à l'extrémité distale (234) du tube intérieur (206).

5. Dispositif (10) selon l'une des revendications 3 ou 4 précédentes, dans lequel les tubes intérieur et extérieur sont positionnés coaxialement de telle sorte que le canal de mise à l'air (240) entoure la surface extérieure (236) du tube intérieur (206).

6. Dispositif (10) selon l'une des revendications 3 à 5 précédentes, dans lequel le boîtier d'adaptateur (202) définit au moins un évent (242) en communication fluidique avec le canal de mise à l'air (240).

7. Dispositif (10) selon l'une des revendications 3 à 6 précédentes, dans lequel le boîtier d'adaptateur (202) comprend un filetage (212).

8. Dispositif (10) selon l'une quelconque des revendications précédentes, dans lequel l'extrémité distale (234) du tube intérieur (206) est située proximalement par rapport à l'extrémité distale (226) du tube extérieur (204).

9. Dispositif (10) selon l'une quelconque des revendications précédentes, dans lequel l'extrémité distale (226) du tube extérieur (204) comporte un bord arrondi.

10. Dispositif selon l'une quelconque des revendications précédentes, comprenant en outre un récipient de stockage (300) pouvant être couplé au corps principal (100) et en communication fluidique avec la cavité interne (112), destiné à recevoir le matériau osseux et/ou cellulaire extrait du patient.

11. Dispositif selon l'une des revendications 3 à 10 précédentes, dans lequel le boîtier d'adaptateur (202) comporte en outre un deuxième épaulement faisant saillie vers l'intérieur (222), distal par rapport au premier épaulement (220), l'extrémité proximale (224) du tube extérieur (204) étant en prise avec le deuxième épaulement faisant saillie vers l'intérieur.

12. Dispositif selon la revendication précédente, dans lequel l'au moins un évent (242) est situé axialement entre les premier et deuxième épaulements.

13. Dispositif selon l'une quelconque des revendications précédentes, dans lequel le tube intérieur (206) est en communication fluidique avec la cavité interne (112).

14. Dispositif selon l'une quelconque des revendications précédentes, dans lequel le canal de mise à l'air est défini entre une partie d'une surface extérieure (236) du tube intérieur (206) et une surface intérieure (230) du tube extérieur (204).
